# EUROPÄISCHE PATENTANMELDUNG

(11) **EP 2 191 854 A2**
(43) Veröffentlichungstag der Anmeldung: **02.06.2010**
(21) Anmeldenummer: 09175461.4
(22) Anmeldetag: 10.11.2009
(51) Int. Cl.: A61L 31/02, A61L 31/14

(54) **Stent mit einer Struktur aus einem biokorrodierbaren Werkstoff und einem gesteuerten Korrosionsverhalten**

(30) Priorität: 01.12.2008 DE 102008044221
(71) Anmelder: Biotronik VI Patent AG, 6341 Baar (CH)
(72) Erfinder: Lootz, Daniel, 18119, Rostock (DE); Block, Bernd, 18055, Rostock (DE)
(74) Vertreter: Lindner-Vogt, Karin L.

(57) **Zusammenfassung**

Die Erfindung betrifft einen Stent mit einer Struktur aus einer biokorrodierbaren Magnesium- oder Wolframlegierung, die aus einer Vielzahl miteinander in Verbindung stehender Stegabschnitte besteht, wobei
(i) die Struktur eine Stützstruktur aus einer Anzahl miteinander in Verbindung stehender erster Stegabschnitte aufweist, die ausgelegt sind, eine nach einer Expansion des Stents die Gefäßwand abstützende oder die mechanische Integrität des Stents bewahrende Funktion für einen vorgebbaren Zeitraum wahrzunehmen; und
(ii) mindestens ein zweiter Stegabschnitt vorhanden ist, der
a) direkt mit einem ausgewählten ersten Stegabschnitt verbunden ist,
b) keine nach der Expansion des Stents die Gefäßwand abstützende oder die mechanische Integrität des Stents bewahrende Funktion für den vorgegebenen Zeitraum wahrnimmt und
c) eine kleinere durchschnittliche Korngröße aufweist als die ersten Stegabschnitte der Stützstruktur aus (i).

## Beschreibung

### Technisches Gebiet

Die Erfindung betrifft einen Stent mit einer Struktur aus einem biokorrodierbaren metallischen Werkstoff und einem gesteuerten Korrosionsverhalten, sowie ein Verfahren zur Herstellung eines solchen Stents.

### Stand der Technik

Die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen.

Stents besitzen einen rohrförmigen Grundkörper durch den der Blutfluss ungehindert weiterläuft und dessen Umfangswandung eine Stützfunktion für die Gefäßwand wahrnimmt. Der Grundkörper ist häufig als gitterartige Struktur verwirklicht, mit einer Vielzahl von einzeln und miteinander verbundenen Stegabschnitten. Weiterhin muss die Designvorgabe für die gitterartige Struktur es erlauben, den Stent in einem komprimierten Zustand mit kleinem Außendurchmesser bis zur zu behandelnden Engstelle des jeweiligen Gefäßes einzuführen und dort beispielsweise mit Hilfe eines Ballonkatheters so weit aufzuweiten, dass das Gefäß den gewünschten, vergrößerten Innendurchmesser aufweist. Um unnötige Gefäßbeschädigungen zu vermeiden, sollte der Stent nach dem Aufweiten und Entfernen des Ballons nicht oder allenfalls in geringem Umfang elastisch zurückfedern, so dass der Stent beim Aufweiten nur wenig über den gewünschten Enddurchmesser hinaus geweitet werden muss. Weitere konstruktive Anforderungen sind eine gleichmäßige Flächenabdeckung und eine Struktur, die eine gewisse Flexibilität in Bezug auf die Längsachse des Stents erlaubt. Konstruktiv lassen sich die einzelnen, eine gitterartige Struktur bildenden Stegabschnitte gliedern in solche mit Stützfunktion für die Gefäßwand und tragender Funktion (d. h. die mechanische Integrität des Implantats gewährender Funktion) und solche ohne Beteiligung an den genannten konstruktiven Aufgaben. Das Geflecht der ersteren Stegabschnitte wird nachfolgend als Tragstruktur bezeichnet. In der Praxis wird der Stent zur Wahrnehmung der genannten konstruktiven Vorgaben zumeist aus einem metallischen Werkstoff geformt.

Neben den mechanischen Eigenschaften eines Stents sollte dieser aus einem biokompatiblen Werkstoff bestehen, um Abstoßungsreaktionen zu minimieren. Derzeit werden bei etwa 70% aller perkutanen Interventionen Stents eingesetzt, in 25% aller Fälle kommt es jedoch zu einer in-Stent Restenose aufgrund eines überschießenden neointimalen Wachstums, das durch eine starke Proliferation der arteriellen glatten Muskelzellen und eine chronische Entzündungsreaktion hervorgerufen wird. Zur Senkung der Restenoseraten werden verschiedene Lösungsansätze verfolgt.

Ein Ansatzpunkt ist die Verwendung biokorrodierbarer Metalllegierungen, denn zumeist ist eine dauerhafte Stützfunktion durch den Stent nicht notwendig; das zunächst geschädigte Gefäß kann sich regenerieren. Dementsprechend wird beispielsweise in DE 197 31 021 A1 vorgeschlagen, medizinische Implantate aus einem metallischen Werkstoff zu formen, dessen Hauptbestandteil ein Element aus der Gruppe Alkalimetalle, Erdalkalimetalle, Zink und Aluminium ist. Als besonders geeignet werden Legierungen auf Basis von Magnesium und Zink beschrieben. Nebenbestandteile der Legierungen können Mangan, Cobalt, Nickel, Chrom, Kupfer, Cadmium, Blei, Zinn, Thorium, Zirkonium, Silber, Gold, Palladium, Platin, Silizium, Calcium, Lithium, Aluminium und Zink sein. Weiterhin ist aus der DE 102 53 634 A1 der Einsatz einer biokorrodierbaren Magnesiumlegierung mit einem Anteil von Magnesium > 90%, Yttrium 3,7 - 5,5%, Seltenerdmetallen 1,5 - 4,4% und Rest < 1% bekannt, die sich insbesondere zur Herstellung einer Endoprothese, z. B. in Form eines selbstexpandierenden oder ballonexpandierbaren Stents, eignet.

Biokorrodierbare Implantate stellen somit einen aussichtsreichen Ansatz zur Minderung der Restenoserate dar. Ein Problem bei der Realisation derartiger Systeme ist das Korrosionsverhalten des Implantats. So sollte eine Fragmentbildung durch den Korrosionsprozess nach Möglichkeit bis zum Einwachsen des Implantats in die Gefäßwand unterbunden sein. Weiterhin sollte die Stützfunktion über den Zeitraum der therapeutischen Vorgabe aufrecht erhalten werden. Die oben genannten konstruktiven Vorgaben lassen eine freie Anpassung des Stentdesigns in Bezug auf sein Korrosionsverhalten nicht zu - es müssen Kompromisse eingegangen werden.

Der vorliegenden Erfindung liegt die Aufgabe zugrunde, die geschilderten Nachteile des Standes der Technik zu überwinden. Insbesondere soll ein Stent mit einem verbesserten Korrosionsverhalten bereitgestellt werden.

### Erfindungsgemäße Lösung

Die Aufgabe wird durch Bereitstellung eines Stents gelöst mit einer Struktur aus einer biokorrodierbaren Magnesium- oder Wolframlegierung, die aus einer Vielzahl miteinander in Verbindung stehender Stegabschnitte besteht, wobei
(i) die Struktur eine Stützstruktur aus einer Anzahl miteinander in Verbindung stehender erster Stegabschnitte aufweist, die ausgelegt sind eine nach einer Expansion des Stents die Gefäßwand abstützende oder die mechanische Integrität des Stents bewahrende Funktion für einen vorgebbaren Zeitraum wahrzunehmen; und
(ii) mindestens ein zweiter Stegabschnitt vorhanden ist, der
   a) direkt mit einem ausgewählten ersten Stegabschnitt verbunden ist,
   b) keine nach der Expansion des Stents die Gefäßwand abstützende oder die mechanische Integrität des Stents bewahrende Funktion für den vorgegebenen Zeitraum wahrnimmt und
   c) eine kleinere durchschnittliche Korngröße aufweist als die ersten Stegabschnitte der Stützstruktur aus (i).

Der Erfindung liegt die Erkenntnis zugrunde, dass eine kleinere durchschnittliche Korngröße in einem metallischen Werkstoff zu einer erhöhten Korrosionsrate in diesem Bereich führt. Die sogenannte Kornfeinung besteht darin, dass z.B. durch lokale Wärmebehandlung ein kleineres, feineres Korn im Gefüge des metallischen Werkstoffs erreicht wird. In einem Körper, der z.B. aus einem einzigen metallischen Werkstoff bestehen kann, können so Bereiche mit unterschiedlicher durchschnittlicher Korngröße und damit mit unterschiedlichen Korrosionseigenschaften erzeugt werden.

Erfindungsgemäß ist nun vorgesehen, diese Unterschiede im Korrosionsverhalten desselben metallischen Werkstoffs in Abhängigkeit seiner Korngröße dazu zu nutzen, das Korrosionsverhalten eines Stent mit einer Struktur aus einer biokorrodierbaren Magnesium- oder Wolframlegierung in einer ersten Phase der Degradation zu beeinflussen. Der genannte Zeitraum beginnt unmittelbar nach der Implantation und endet zu einem vorgebbaren Zeitpunkt, der den therapeutischen Vorgaben und Anforderungen an die Sicherheit entspricht. Vorzugsweise erstreckt sich dieser Zeitraum über 2 bis 6 Wochen unmittelbar nach der Implantation. In der Regel ist der Stent innerhalb dieses Zeitraums in die Gefäßwand eingewachsen und diese ist soweit regeneriert, dass eine weitere Stützfunktion nicht mehr notwendig ist.

Eine Tragstruktur im Sinne der Erfindung fasst die Stegabschnitte zusammen, die eine Stützfunktion für die Gefäßwand über den vorgegebenen Zeitraum und die Konstruktion tragende Funktion (d. h. die mechanische Integrität des Implantats bewahrende Funktion) über den vorgegebenen Zeitraum wahrnehmen. Dies sind insbesondere die Stegabschnitte, ohne die die Stützfunktion und tragende Funktion nicht mehr den Erfordernissen am Implantationsort genügt.

Durch eine gezielte Wärmebehandlung werden nun Stegabschnitte mit verringerter Korngröße in bestimmten Bereichen der Tragstruktur bereitgestellt. Diese besonderen Stegabschnitte sind so geschaffen, dass ihre durchschnittliche Korngröße kleiner ist als die durchschnittliche Korngröße des jeweiligen Stegabschnittes der Tragstruktur, mit dem sie verbunden sind. Durch Erzeugung von kleineren durchschnittlichen Korngrößen werden mehr Korngrenzen pro mm² bzw. mehr Korngrenzflächen pro mm³ metallischen Werkstoff bereitgestellt, die Angriffspunkte für einen Korrosionsvorgang darstellen können. Der Stegabschnitt mit kleinerer durchschnittlicher Korngröße weist daher eine erhöhte Korrosionsrate auf, wird also zuerst korrodiert und bildet somit einen definierten Nukleationspunkt für den Beginn der Korrosion des erfindungsgemäßen Stents. Durch geeignete Auswahl der Bereiche mit kleinerer Korngröße kann also kontrolliert festgelegt werden, wo im Stent die Korrosion beginnen und von wo aus sie sich über die Zeit ausbreiten soll. Dadurch wird der mit dem Stegabschnitt mit kleinerer Korngröße verbundene Stegabschnitt der Tragstruktur temporär stabilisiert. Über die Auswahl der Anzahl, Ausdehnung und der Anordnung der Stegabschnitte mit kleinerer Korngröße im Stent kann eine Dauer des stabilisierenden Effektes beeinflusst werden und zwar mit der Maßgabe, dass mindestens der oben genannte vorgegebene Zeitraum sichergestellt ist. Natürlich hat der Stegabschnitt mit kleinerer durchschnittlicher Korngröße nicht selbst eine tragende Funktion wahrzunehmen oder zur Aufrechterhaltung der mechanischen Integrität des Implantats zu dienen. Ein besonderer Vorteil des erfindungsgemäßen Konzepts liegt darin, dass für das gesamte Implantat der gleiche biodegradierbare metallische Werkstoff verwendet wird und eine Feinabstimmung der Werkstoffeigenschaften hinsichtlich des Korrosionsverhaltens durch eine unterschiedliche Wärmebehandlung des Materials in bestimmten Stegabschnitten erreicht wird. Geeignete Unterschiede in der mittleren Korngröße zwischen den ersten Stegabschnitten und dem mindestens einen zweiten Stegabschnitt ergeben sich für den Fachmann ohne Schwierigkeiten, lediglich unter Zuhilfenahme von Routineversuchen und der bekannten Hall-Petch-Beziehung.

Einerseits möchte man bei Stents frühzeitig eine hohe Biegeflexibilität erreichen, jedoch andererseits eine möglichst lange radiale Festigkeit oder Integrität erhalten. Dabei sind die Verbinderstrukturen, die die Stentelemente mit radialer Stützfunktion miteinander verbinden nur für die Stentmontage und -applikation erforderlich. Nach Implantation und Stentintegration in die Gefäßwand werden dann nur noch die Ringelemente benötigt, die die tragende Funktion zum Offenhalten des Gefäßes übernehmen. Die erfindungsgemäßen Stents eigenen sich besonders für solche Anwendungen, wenn z.B. einer oder mehrere der Verbinder als zweite Stegabschnitte mit kleinerer durchschnittlicher Korngröße ausgebildet sind.

Andererseits möchte man bei wachsenden Gefäßen (z.B. Stents für pädiatrische Anwendungen) deren Wachstum durch Ringelemente eines Stents nicht behindern. Hier gewährleisten definierte Korrosions- oder Degradationsstellen im Ringelement des Stents einen Zerfall des Stents, der den restlichen Stentelementen noch eine ausreichende Stützfunktion erhält, ohne das radiale Wachstum des Gefäßes im Wesentlichen zu behindern. Hierfür eignen sich die erfindungsgemäßen Stents besonders, da ausgewählte "Sollbruchstellen" im Ringelement des Stents gezielt festgelegt und erzeugt werden können, ohne dass auf eine Herstellung des Stents aus unterschiedlichen Materialien zurückgegriffen werden muss.

Die Erzeugung einer kleineren durchschnittlichen Korngröße im zweiten Stegabschnitt kann auf verschiedenen Wegen erreicht werden. Erfindungsgemäß ist die feinere Kornstruktur des mindestens einen zweiten Stegabschnitts durch lokale Wärmebehandlung herstellbar. Grundsätzlich eignet sich jedes Verfahren zur lokalen Wärmebehandlung, vorausgesetzt es erlaubt eine Behandlung der ausgewählten zweiten Stegabschnitte mit einer Temperatur, die höher ist, als die Temperatur, der die ersten Stegabschnitte ausgesetzt sind. Dem Fachmann sind geeignete Formen der lokalen Wärmebehandlung bekannt bzw. er hat keine Schwierigkeiten im Rahmen von Routineversuchen bekannte Verfahren zur lokalen Wärmebehandlung so anzupassen, dass sie auf Stents anwendbar sind und zu den erfindungsgemäßen Stents führen. Bevorzugt ist die lokale Wärmebehandlung z.B. ausgewählt aus einer Behandlung des Stents mit einem Laserstrahl oder mit einem Elektronenstrahl.

In einer bevorzugten Ausführungsform des erfindungsgemäßen Stents werden vorher definierte Abschnitte des mindestens einen zweiten Stegabschnitts gezielt der lokalen Wärmebehandlung zugeführt.

Die Erzeugung einer kleineren durchschnittlichen Korngröße im mindestens einen zweiten Stegabschnitt kann in jedem Stadium der Stentherstellung und Stentbereitstellung bis zum Einsetzen des Stents erfolgen. Es kann also der fertig vorbereitete, gecrimpte Stent ebenso einer lokalen Wärmebehandlung unterzogen werden, wie Stent-Vorläuferprodukte. Der Stent kann dabei aus einem Rohr geschnitten werden. Anschließend werden gezielt Bereiche für die lokale Wärmebehandlung ausgewählt und einer entsprechenden Behandlung unterzogen. Alternativ kann z.B. auch das Halbzeug, also z.B. das noch nicht abschließend zurechtgeschnittene Rohr, an solchen Orten, die später einen zweiten Stegabschnitt i. S. d. Erfindung darstellen sollen, einer lokalen Wärmebehandlung unterzogen werden.

Vorzugsweise ist der biokorrodierbare metallische Werkstoff eine biokorrodierbare Magnesium- oder Wolframlegierung; insbesondere ist der biokorrodierbare metallische Werkstoff eine Magnesiumlegierung. Unter Legierung wird vorliegend ein metallisches Gefüge verstanden, deren Hauptkomponente Magnesium oder Wolfram ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%. Bevorzugt enthält die Legierung des erfindungsgemäßen Stent im Wesentlichen kein Eisen.

Ist der Werkstoff eine Magnesiumlegierung, so enthält diese vorzugsweise Yttrium und weitere Seltenerdmetalle, da sich eine derartige Legierung aufgrund ihrer physikochemischen Eigenschaften und hohen Biokompatibilität, insbesondere auch seiner Abbauprodukte, auszeichnet.

Besonders bevorzugt wird eine Magnesiumlegierung der Zusammensetzung Seltenerdmetalle 5,2 - 9,9 Gew.%, davon Yttrium 3,7 - 5,5 Gew.%, und Rest < 1 Gew.%, wobei Magnesium den auf 100 Gew.% fehlenden Anteil an der Legierung einnimmt, eingesetzt. Diese Magnesiumlegierung bestätigte bereits experimentell und in ersten klinischen Versuchen ihre besondere Eignung, d. h. zeigt eine hohe Biokompatibilität, günstige Verarbeitungseigenschaften, gute mechanische Kennwerte und ein für die Einsatzzwecke adäquates Korrosionsverhalten. Unter der Sammelbezeichnung "Seltenerdmetalle" werden vorliegend Scandium (21), Yttrium (39), Lanthan (57) und die 14 auf Lanthan (57) folgenden Elemente, nämlich Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71), verstanden.

Die Legierungen der Elemente Magnesium oder Wolfram sind so in ihrer Zusammensetzung zu wählen, dass sie biokorrodierbar sind. Als biokorrodierbar im Sinne der Erfindung werden Legierungen bezeichnet, bei denen in physiologischer Umgebung ein Abbau stattfindet, der letztendlich dazu führt, dass das gesamte Implantat oder der aus dem Werkstoff gebildete Teil des Implantates seine mechanische Integrität verliert. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/l, CaCl 0,2 g/l, KCI 0,4 g/l, MgSO₄ 0,1 g/l, NaHCO₃ 2,2 g/l, Na₂HPO₄ 0,126 g/l, NaH₂PO₄ 0,026 g/I). Eine Probe der zu untersuchenden Legierung wird dazu in einem verschlossenen Probenbehälter mit einer definierten Menge des Prüfmediums bei 37°C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Der Begriff Korrosion bezieht sich vorliegend auf die Reaktion des metallischen Werkstoffes mit seiner Umgebung, wobei eine messbare Veränderung des Werkstoffs bewirkt wird, die - bei Einsatz des Werkstoffs in einem Bauteil - zu einer Beeinträchtigung der Funktion des Bauteils führt. Ein Korrosionssystem besteht aus dem korrodierenden metallischen Werkstoff sowie einem flüssigen Korrosionsmedium, das in seiner Zusammensetzung die Verhältnisse in physiologischer Umgebung nachstellt oder ein physiologisches Medium, insbesondere Blut ist. Werkstoffseitig beeinflusst die Korrosion Faktoren, wie die Zusammensetzung und Vorbehandlung der Legierung, mikro- und submikroskopische Inhomogenitäten, Randzoneneigenschaften, Temperatur- und Spannungszustand und insbesondere die Zusammensetzung einer die Oberfläche bedeckenden Schicht. Auf Seiten des Mediums wird der Korrosionsprozess durch Leitfähigkeit, Temperatur, Temperaturgradienten, Azidität, Volumen-Oberflächen-Verhältnis, Konzentrationsunterschied sowie Strömungsgeschwindigkeit beeinflusst.

In einem weiteren Aspekt bezieht sich die Erfindung auf ein Verfahren zur Herstellung eines erfindungsgemäßen Stents, umfassend die Schritte
a) Bereitstellen eines Stents oder eines Stent-Vorläuferproduktes mit mindestens einem festgelegten ersten und zweiten Stegabschnitt,
b) lokale Behandlung des festgelegten mindestens einen zweiten Stegabschnitts mit Wärme einer Temperatur, die höher ist als die Temperatur, der der mindestens eine erste Stegabschnitt ausgesetzt ist.

In diesem Verfahren kommen Stents oder Stent-Vorläuferprodukte mit einer Struktur aus einer biokorrodierbaren Magnesium- oder Wolframlegierung zum Einsatz.

Das erfindungsgemäße Verfahren kann in jedem Stadium der Stentherstellung und Stentbereitstellung bis zum Einsetzen des Stents erfolgen. Es kann also der fertig vorbereitete, gecrimpte Stent ebenso einer lokalen Wärmebehandlung unterzogen werden, wie Stent-Vorläuferprodukte. Der Stent kann dabei aus einem Rohr geschnitten werden. Anschließend werden gezielt Bereiche für die lokale Wärmebehandlung ausgewählt und einer entsprechenden Behandlung unterzogen. Alternativ kann z.B. auch das Halbzeug, also z.B. das noch nicht abschließend zurechtgeschnittene Rohr, an solchen Orten, die später einen zweiten Stegabschnitt i. S. d. Erfindung darstellen sollen, einer lokalen Wärmebehandlung unterzogen werden.

Grundsätzlich eignet sich jedes Verfahren zur lokalen Wärmebehandlung, vorausgesetzt es erlaubt eine lokale Behandlung des festgelegten mindestens einen zweiten Stegabschnitts mit Wärme einer Temperatur, die höher ist als die Temperatur, der der mindestens eine erste Stegabschnitt ausgesetzt ist. Dem Fachmann sind geeignete Formen der lokalen Wärmebehandlung bekannt bzw. er hat keine Schwierigkeiten im Rahmen von Routineversuchen bekannte Verfahren zur lokalen Wärmebehandlung so anzupassen, dass sie auf Stents anwendbar sind. Bevorzugt ist die lokale Wärmebehandlung z.B. ausgewählt aus einer Behandlung des Stents mit einem Laserstrahl oder mit einem Elektronenstrahl.

## Patentansprüche

1. Stent mit einer Struktur aus einer biokorrodierbaren Magnesium- oder Wolframlegierung, die aus einer Vielzahl miteinander in Verbindung stehender Stegabschnitte besteht, wobei
(i) die Struktur eine Stützstruktur aus einer Anzahl miteinander in Verbindung stehender erster Stegabschnitte aufweist, die ausgelegt sind, eine nach einer Expansion des Stents die Gefäßwand abstützende oder die mechanische Integrität des Stents bewahrende Funktion für einen vorgebbaren Zeitraum wahrzunehmen; und
(ii) mindestens ein zweiter Stegabschnitt vorhanden ist, der
a) direkt mit einem ausgewählten ersten Stegabschnitt verbunden ist,
b) keine nach der Expansion des Stents die Gefäßwand abstützende oder die mechanische Integrität des Stents bewahrende Funktion für den vorgegebenen Zeitraum wahrnimmt und
c) eine kleinere durchschnittliche Korngröße aufweist als die ersten Stegabschnitte der Stützstruktur aus (i).

2. Stent nach Anspruch 1, **dadurch gekennzeichnet, dass** die kleinere durchschnittliche Korngröße des mindestens einen zweiten Stegabschnitts durch lokale Wärmebehandlung herstellbar ist.

3. Stent nach Anspruch 2, **dadurch charakterisiert, dass** die lokale Wärmebehandlung ausgewählt ist aus der Behandlung mit einem Laserstrahl oder mit einem Elektronenstrahl.

4. Stent nach einem der vorhergehenden Ansprüche, **dadurch charakterisiert, dass** vorher definierte Abschnitte des mindestens einen zweiten Stegabschnitts gezielt der lokalen Wärmebehandlung zugeführt werden.

5. Verfahren zur Herstellung eines Stents nach einem der Ansprüche 1 bis 4, umfassend die Schritte:
a) Bereitstellen eines Stents oder eines Stent-Vorläuferproduktes mit mindestens einem festgelegten ersten und zweiten Stegabschnitt,
b) lokale Behandlung des festgelegten mindestens einen zweiten Stegabschnitts mit Wärme einer Temperatur, die höher ist als die Temperatur, der der mindestens eine erste Stegabschnitt ausgesetzt ist.

6. Verfahren nach Anspruch 5, wobei in Schritt b) ein Laserstrahl zum Einsatz kommt.

7. Verfahren nach Anspruch 5, wobei in Schritt b) ein Elektronenstrahl zum Einsatz kommt.
